# EUROPÄISCHE PATENTSCHRIFT

(11) **EP 3 485 079 B1**
(45) Veröffentlichungstag und Bekanntmachung des Hinweises auf die Patenterteilung: **18.10.2023**
(21) Anmeldenummer: 17739244.6
(22) Anmeldetag: 11.07.2017
(51) Int. Cl.: D06F 34/18, G01N 33/543, D06F 103/02, D06F 105/42, D06F 105/52, D06F 33/32

(54) **VERFAHREN UND VORRICHTUNG ZUR ERMITTLUNG VON VERUNREINIGUNGEN**
METHOD AND DEVICE FOR DETERMINING SOILING
PROCÉDÉ ET DISPOSITIF PERMETTANT DE DÉTERMINER DES SALISSURES

(30) Priorität: 15.07.2016 DE 102016212978
(43) Veröffentlichungstag der Anmeldung: 22.05.2019
(73) Patentinhaber: Henkel AG & Co. KGaA, 40589 Düsseldorf (DE)
(72) Erfinder: KESSLER, Arnd, 40789 Monheim am Rhein (DE); NITSCH, Christian, 40591 Düsseldorf (DE); ZÜCHNER, Lars, 40764 Langenfeld (DE); WAWER, Georg, 1040 Wien (AT); MÜLLER, Alexander, 40789 Monheim (DE); WICK, Wolfgang, 41542 Dormagen (DE)
(86) Internationale Anmeldenummer: PCT/EP2017/067338
(87) Internationale Veröffentlichungsnummer: WO 2018/011174

(56) Entgegenhaltungen:
- EP-A1- 0 725 181
- EP-A2- 1 990 461
- WO-A1-2004/058038
- DE-A1- 19 855 503
- DE-T5-112013 004 547

## Beschreibung

### Gebiet der Erfindung

Die Erfindung betrifft Verfahren und Vorrichtungen, welche bei der Ermittlung von Verunreinigungen eingesetzt werden können.

### Hintergrund der Erfindung

Verunreinigungen auf einer Textilie, etwa Kleidungsstücke, Gardinen oder Bettzeug, sind oft schwierig zu identifizieren. Verunreinigungen können dabei nicht nur die Ästhetik der Textilien beeinflussen, sondern auch ein hygienisches Problem für den Benutzer der Textilie darstellen. Viele Verunreinigungen sind zwar mit dem Auge leicht auszumachen, jedoch ist dem Benutzer der Textilie oft unklar, welche Zusammensetzung bzw. welchen Ursprung die Verunreinigung hat. In manchen Fällen ist dem Benutzer beispielsweise nicht bewusst, dass die Textilie bei einem Missgeschick gerade verunreinigt wird. Die Verunreinigung fällt dem Benutzer dann erst zu einem späteren Zeitpunkt auf, wobei sich die Ursache und die Zusammensetzung der Verunreinigung dem Benutzer verschließen. Verunreinigungen mit verschiedenen Zusammensetzungen können für das Auge auch eine sehr ähnliche Erscheinung bieten, beispielsweise können Blutflecken und Tomatenflecken insbesondere nach einem gewissen Zeitraum mit dem Auge nicht mehr zu unterscheiden sein.

Hierbei ist es wünschenswert für den Benutzer, einen Hinweis auf die Zusammensetzung der Verunreinigung zu erlangen. Insbesondere lassen sich damit hygienische Probleme mit den Textilien identifizieren oder ausschließen. Zudem ist es zumeist von Interesse, die Verunreinigung durch einen Reinigungsvorgang wieder zu entfernen. Solche Reinigungsvorgänge können durch Hinweise auf die Zusammensetzung der Verunreinigung erheblich erleichtert oder überhaupt erst ermöglicht werden.

Dokument DE19855503 A1 beschreibt ein Verfahren zur Anpassung des Reinigungsprogramms einer Waschmaschine basierend auf der Identifizierung von Textileigenschaften mit Hilfe eines Spektrometers. Dokument DE112013004547 T5 beschreibt ein Verfahren zur Verbesserung der Reinigungsleistung durch Messung des Gewichts von Waschgut und der Eigenschaften des Waschwassers mit Hilfe eines Trübungs- oder Leitfähigkeitssensors. Dokument EP1990461 A2 beschreibt ein Verfahren zur Anpassung des Reinigungsprogramms eines Geräts basierend auf Fleckeneigenschaften gemessen mittels eines optischen Sensors.

Aus der WO 01/96645 A2 ist beispielsweise ein Verfahren zur Optimierung eines Verbrauchergeräts bekannt. Hierzu kann beispielsweise eine Waschmaschine einen Sensor zur Detektion einer Eigenschaft eines verschmutzten Artikels aufweisen. Hiermit soll die Reinigung des Artikels verbessert werden. Dabei wird auf ph-Sensoren, Leitfähigkeitssensoren, Wasserhärtesensoren, Trübheitssensoren, Temperatursensoren, Calcium-Ionen-Sensoren und Redox-Potential-Sensoren hingewiesen. Nachteilig ist jedoch, dass derartige Sensoren in Bezug auf eine Verunreinigung unter Umständen keine ausreichenden Informationen oder unzuverlässige Ergebnisse über die Verschmutzung liefern.

### Allgemeine Beschreibung einiger beispielhafter Ausführungsformen der Erfindung

Vor dem Hintergrund des dargestellten Stands der Technik ist es somit Aufgabe der Erfindung, die beschriebenen Probleme zumindest teilweise zu verringern oder zu vermeiden, das heißt auf zuverlässige Weise Hinweise auf die Zusammensetzung von Verunreinigungen auf Textilien zur Verfügung zu stellen. Die vorgeschlagenen Verfahren und Vorrichtungen sollen insbesondere auch benutzerfreundlich im Haushalt verwendet werden können.

Gemäß einem ersten Aspekt der Erfindung wird ein Verfahren offenbart, durchgeführt von einer Vorrichtung, umfassend: Erhalten eines Messergebnisses zumindest eines Biosensors repräsentativ für eine Verunreinigung einer Textilie, wobei der zumindest eine Biosensor zumindest ein biologisch aktives immobilisiertes System aufweist, wobei das biologisch aktive immobilisierte System auf Antikörpern, Enzymen, Organellen, Mikroorganismen, Nukleinsäuren, Zellen und/oder Gewebe basiert und, ein Signalumwandler und/oder ein elektronisches System aufweist; wobei der Signalumwandler eine physikochemische Veränderung durch eine Wechselwirkung zwischen dem biologischen aktiven immobilisierten System und einem Analyt bestimmt; Ermitteln mindestens einer von der Zusammensetzung der Verunreinigung abhängigen Ausgangsgröße auf Basis des Messergebnisses; und Ausgeben oder Auslösen eines Ausgebens der mindestens einen Ausgangsgröße.

Gemäß einem zweiten Aspekt der Erfindung wird eine Vorrichtung beschrieben, umfassend zumindest einen Prozessor und zumindest einen Speicher mit Computerprogrammcode, welche dazu eingerichtet sind, ein Verfahren nach dem ersten Aspekt durchzuführen und/oder zu steuern.

Unter einer Verunreinigung wird insbesondere eine Ansammlung von Fremdstoff auf dem Material der Textilie oder eine Verfärbung der Oberfläche der Textilie verstanden, insbesondere in Form eines Flecks, Schmutz oder Unsauberkeiten. Beispielsweise befinden sich auf der Oberfläche Partikel wie Staub, Spuren von Flüssigkeiten, Farbstoffe oder fettige Rückstände. Weiter können auch nicht fixierte Textilfarbstoffe in dem Material der Textilie eingebracht worden sein, wobei sich die nicht fixierten Textilfarbstoffe aus dem Material lösen können, beispielsweise bei einem Reinigungsvorgang wie einem Waschen. Unter einer Verunreinigung können auch solche herausgelösten Textilbestandteile wie Textilfarbstoffe verstanden werden.

Unter einer Textilie werden insbesondere Kleidungsstücke, Gardinen oder Bettzeug verstanden. Kleidungsstücke und Bettzeug umfassen beispielsweise Hemden, T-Shirts, Kleider, Jacken, Pullover, Hosen, Decken, Abdeckungen und Bezüge. Die Textilien können verschiedene Materialien umfassen, beispielsweise Naturfasern, Chemiefasern oder auch weitere Materialien wie Leder.

Es hat sich gezeigt, dass das Messergebnis eines Biosensors dazu verwendet werden kann, um zuverlässig Informationen über die Zusammensetzung einer Verunreinigung einer Textilie zu erhalten und daraus eine Ausgangsgröße zu ermitteln. Es hat sich gezeigt, dass gerade bei der Gruppe von Verunreinigungen, welche regelmäßig auf Textilien auftreten, Biosensoren besonders voreilhaft, das heißt insbesondere zuverlässig einsetzbar sind. Durch den einen oder die mehrere Biosensoren kann im Ergebnis ein Fingerabdruck der Verunreinigung erhalten werden.

Unter einem Biosensor oder auch biochemischen Sensor wird ein Sensor verstanden, welcher ein immobilisiertes biologisch aktives System zur Erzeugung des Messergebnisses verwendet. Dabei kann eine biochemische (beispielsweise enzymatische) Reaktion genutzt werden. Ebenfalls weist der Biosensor elektronische Komponenten, wie einen Signalverstärker auf. Unter einem Biosensor wird insbesondere ein Biosensor gemäß der Definition der IUPAC (International Union of Pure and Applied Chemistry) verstanden.

Der Biosensor kann dabei beispielsweise dazu eingerichtet sein, eine oder mehrere verschiedene Substanzen zu erkennen oder zu bestimmen. Für diese Erkennung der zu bestimmenden Substanzen können Biosensoren biologische Systeme auf unterschiedlich hohem Integrationsniveau verwenden.

Das biologisch aktive System des Biosensors tritt in (mittelbare oder unmittelbare) Wechselwirkung mit einem Analyten. Der Analyt kann beispielsweise die Verunreinigung oder eine Probe hiervon sein. Ebenfalls kann der Analyt weitere Substanzen, beispielsweise ein Lösungsmittel, beispielsweise Wasser, umfassen. Durch die Wechselwirkung zwischen dem biologischen System und dem Analyt kann es insbesondere zu physikochemischen Veränderungen kommen, wie z. B. Veränderungen einer Schichtdicke, eines Brechungsindizes, einer Lichtabsorption oder einer elektrischen Ladung, um nur einige Beispiele zu nennen. Diese physikochemischen Veränderungen werden anschließend bestimmt, sodass ein Messergebnis erhalten werden kann. Nach dem Messvorgang wird bevorzugt der Ausgangszustand des Biosensors wiederhergestellt, sodass dieser erneut verwendet werden kann.

Darunter, dass das Messergebnisses repräsentativ für eine Verunreinigung einer Textilie ist, wird insbesondere verstanden, dass die Verunreinigung und insbesondere die Zusammensetzung der Verunreinigung zumindest teilweise das Messergebnis beeinflusst. Da die Zusammensetzung der Verunreinigung das resultierende Messergebnis beeinflusst, kann aus der Messgröße eine Ausgangsgröße ermittelt werden, welche von der Zusammensetzung der Verunreinigung abhängig ist.

Zur Ermittlung der Ausgangsgröße auf Basis des Messergebnisses kann beispielsweise berücksichtigt werden, ob das Messergebnis einen bestimmten Wert aufweist, wie hoch ein Wert des Messergebnisses ist. Ebenfalls kann jedoch ein zeitlicher Verlauf des Messergebnisses berücksichtigt werden, beispielswiese ein Signalanstieg und/oder Signalabfall des Messergebnisses.

Das gemäß dem ersten Aspekt erhaltene Messergebnis ist bevorzugt repräsentativ für eine Verunreinigung auf einer Oberfläche einer Textilie.

Bei der mindestens einen Ausgangsgröße kann es sich insbesondere um einen oder mehrere Werte handeln, welche repräsentativ für die chemische Zusammensetzung der Verunreinigung, für den Verunreinigungsgrad und/oder für die räumliche Verteilung der Verunreinigung sind. Beispielsweise umfasst die mindestens eine Ausgangsgröße mindestens einen Wert für das Vorkommen und/oder die Konzentration eines chemischen Elements oder einer chemischen Verbindung, die Menge der Verunreinigung oder die Flächenausdehnung der Verunreinigung.

Durch das Ausgeben oder das Auslösen eines Ausgebens der mindestens einen Ausgangsgröße kann somit dem Benutzer eine Information zur Zusammensetzung der Verunreinigung der Textilie bereitgestellt werden, welche vorteilhafterweise zur Identifikation der Verunreinigung beiträgt. Dem Benutzer können beispielsweise Informationen über die chemische Zusammensetzung bzw. über das Vorkommen einzelner Elemente oder Verbindungen zur Verfügung gestellt werden. Insbesondere durch eine Klassifizierung kann mit der mindestens einen Ausgangsgröße eine weitergehende Information bereitgestellt werden, beispielsweise ob die Verunreinigung Gehalte an bestimmten organischen oder anorganischen Komponenten enthält, etwa Farbstoffe oder Lipide, Polysaccharide oder Proteine und ggf. welchen Ursprung die Verunreinigung hat. Beispielsweise kann die mindestens eine Ausgangsgröße dem Benutzer Informationen über eine mögliche hygienische Bedenklichkeit der Verunreinigung geben.

Das Verfahren gemäß dem ersten Aspekt bzw. die Vorrichtung gemäß dem zweiten Aspekt ermöglicht es somit insbesondere, dem Benutzer die Identifizierung der Zusammensetzung bzw. des Ursprungs der Verunreinigung zu erleichtern. Ist beispielsweise eine Verunreinigung mit dem Auge nicht zu identifizieren, kann über das Verfahren bzw. die Vorrichtung mindestens eine von der Zusammensetzung der Verunreinigung abhängige Ausgangsgröße ermittelt werden. Die Ausgangsgröße kann es dem Benutzer beispielsweise ermöglichen, zwischen verschiedenen Zusammensetzungen von Verunreinigungen, welche für das Auge gleiche Erscheinungsbilder bieten, zu unterscheiden.

Gemäß einer Ausgestaltung des Verfahrens gemäß dem ersten Aspekt ist die Verunreinigung eine lokalisierbare oder eine nicht lokalisierbare Verunreinigung.

Unter einer lokalisierbaren Verschmutzung wird insbesondere eine lokal begrenzte und insbesondere sichtbare Verunreinigung, beispielsweise ein Fleck, verstanden. Unter einer nicht lokalisierbaren Verschmutzung wird beispielsweise eine insbesondere nicht sichtbare Verunreinigung der Textilie, beispielsweise mit Staub, (Haut-)fett oder Schweiß verstanden. Unter einer nicht lokalisierbaren Verunreinigung soll ebenfalls ein nicht (ausreichend) fixierter Farbstoff der Textilie oder ein Farbstoff, der durch einen Waschvorgang herausgelöst wird, verstanden werden.

Gewöhnlich soll die Verunreinigung durch einen Reinigungsvorgang wieder entfernt werden. In einer Ausgestaltung des Verfahrens gemäß dem ersten Aspekt umfasst die mindestens eine Ausgangsgröße mindestens einen Parameter einer Reinigungsstrategie der Textilie. Dem Benutzer kann somit eine Empfehlung über einen in Bezug auf die vorliegende Verunreinigung optimalen Reinigungsvorgang gegeben werden.

Für eine Verunreinigung kann über das von der Zusammensetzung der Verunreinigung abhängige Messergebnis ein Rückschluss auf Parameter der Reinigungsstrategie gezogen werden. Hierbei kann der mindestens eine Parameter der Reinigungsstrategie indirekt aus einer weiteren Ausgangsgröße bestimmt werden, beispielsweise wird zunächst eine für die Zusammensetzung der Verunreinigung repräsentative Ausgangsgröße bestimmt und aus dieser für die Zusammensetzung der Verunreinigung repräsentativen Ausgangsgröße mindestens ein Parameter der Reinigungsstrategie ermittelt. Auch kann mindestens ein Parameter direkt aus dem Messergebnis ermittelt werden, beispielsweise mittels einer Klassifizierung über hinterlegte Messergebnisse und über den Klassen zugeordneten Reinigungsstrategien.

Insbesondere wenn ein Benutzer der Textilie die Verunreinigung nicht mit dem Auge identifizieren kann und es daher unklar ist, wie die Verunreinigung wieder zu entfernen ist, kann mit dem Verfahren gemäß dem ersten Aspekt bzw. der Vorrichtung gemäß dem zweiten Aspekt eine Empfehlung über eine optimale Reinigungsstrategie bereitgestellt werden. Beispielsweise kann es dem Benutzer unklar sein, ob eine Verunreinigung Lipide oder bestimmte Farbstoffe enthält, welche über gewöhnlicherweise angewandte Reinigungsstrategien nicht zuverlässig entfernt werden können. Über die von der Zusammensetzung der Verunreinigung abhängige, im Rahmen des Verfahrens ermittelte Reinigungsstrategie können mit der Identifikation entsprechender Inhaltsstoffe der Verunreinigung eine Empfehlung über eine auf die individuelle Zusammensetzung angepasste Reinigungsstrategie getroffen werden. Im Ergebnis kann die Entfernung der Verunreinigung stark vereinfacht und deutlich zuverlässiger gestaltet werden.

Denkbar ist es ebenfalls, dass als Verunreinigung aus dem Material der Textilie gelöste Stoffe, beispielsweise nicht fixierte Textilfarbstoffe über die mindestens eine Ausgangsgröße erfasst werden. Dies geschieht insbesondere während der Durchführung einer Reinigungsstrategie, so dass dem Benutzer damit ein Rückschluss auf die Effektivität der Reinigungsstrategie gegeben wird. Beispielsweise kann der Benutzer erkennen, ob sich eine zu große Menge von Textilfarbstoffen aus dem Material der Textilie löst, womit der Benutzer eine Anregung erhält, die Reinigungsstrategie zu verändern und ggf. im Hinblick auf den Textilfarbstoff schonender zu gestalten. Ebenso kann eine Entfärbung einer Textilie beabsichtigt sein und über die mindestens eine Ausgangsgröße ein Rückschluss auf den Grad der Entfärbung durch eine Reinigungsstrategie gezogen werden.

Insbesondere repräsentiert der mindestens eine Parameter der Reinigungsstrategie eine Reinigungsmittelart, eine Reinigungsmittelmenge, eine Reinigungstemperatur, einen Reinigungsvorrichtungstyp, Einstellungen einer Reinigungsvorrichtung oder Kombinationen hiervon.

Reinigungsmittel werden beispielsweise im Haushalt für die Reinigung unterschiedlicher Objekte eingesetzt. Beispielsweise wird für Waschmaschinen ein Reinigungsmittel, zum Beispiel ein Waschmittel, für die Reinigung von Textilien eingesetzt. Unter einem Reinigungsmittel sollen jedoch ebenfalls auch Reinigungshilfsmittel oder Reinigungszusatzmittel, wie beispielsweise ein Bleichzusatzmittel, ein Weichspüler oder Wäschestärke, verstanden werden. Ein Reinigungsmittel kann zudem eine Flüssigkeit, ein disperses System, zum Beispiel ein Gel oder Schaum, oder ein Feststoff, insbesondere ein Tab, Pulver oder Granulat, sein.

Ein Reinigungsmittel kann beispielsweise eine oder mehrere Komponenten aus der Gruppe von Komponenten umfassend Tenside, Alkalien, Builder, Vergrauungsinhibatoren, optische Aufheller, Enzyme, Bleichmittel, Soil-Release-Polymere, Füller, Weichmacher, Duftstoffe, Farbstoffe, Pflegestoffe, Säuren, Stärke, Isomalt, Zucker, Zellulose, Zellulosederivate, Carboxymethylcellulose, Polyetherimid, Silikonderivate und/oder Polymethylimine aufweisen.

Ein Reinigungsmittel kann ferner einen oder mehrere weitere Bestandteile umfassen. Diese Bestandteile schließen ein, sind aber nicht beschränkt auf die Gruppe bestehend aus Bleichaktivatoren, Komplexbildnern, Gerüststoffen, Elektrolyten, nichtwässrigen Lösungsmitteln, pH-Stellmitteln, Parfümträgern, Fluoreszenzmitteln, Hydrotropen, Silikonölen, Bentoniten, Antiredepositionsmitteln, Einlaufverhinderern, Knitterschutzmitteln, Farbübertragungsinhibitoren, , antimikrobiellen Wirkstoffen, Germiziden, Fungiziden, Antioxidantien, Konservierungsmitteln, Korrosionsinhibitoren, Antistatika, Bittermitteln, Bügelhilfsmitteln, Phobier- oder Imprägniermitteln, Quell- oder Schiebefestmitteln und/oder UV-Absorbern.

Der mindestens eine Parameter der Reinigungsstrategie kann die Reinigungsmittelart repräsentieren und somit indikativ für die Zusammensetzung des Reinigungsmittels sein. Wenn beispielsweise ein gewisser Anteil Farbstoffe in der Zusammensetzung der Verunreinigung enthalten sind, kann dem Benutzer die Verwendung von bestimmten Bleichzusatzmitteln empfohlen werden. Sind beispielsweise gewisse Gehalte an Lipiden in der Zusammensetzung der Verunreinigung vorhanden, kann die Verwendung von spezifischen Tensiden und/oder Lipasen in der empfohlenen Reinigungsstrategie enthalten sein.

Der mindestens eine Parameter kann die Reinigungsmittelmenge repräsentieren und insbesondere eine absolute Menge des Reinigungsmittels angeben. Ebenso kann eine relative Menge des Reinigungsmittels mittels des mindestens einen Parameters angezeigt werden, beispielsweise bezogen auf die Masse der zu reinigenden Textilien bzw. ein Flottenverhältnis oder eine Reinigungsmittelmenge bezogen auf ein zur Reinigung einzusetzendes Wasservolumen. Über das von der Zusammensetzung der Verunreinigung abhängige Messergebnis kann somit eine Reinigungsmittelart und/oder eine Reinigungsmittelmenge ermittelt werden, welche eine optimale Entfernung der Verunreinigung gewährleistet.

Mit einem für die Reinigungstemperatur repräsentativen Parameter kann eine für die bestimmte Zusammensetzung der Verunreinigung optimale Temperatur zur Entfernung der Verunreinigung angeben werden, insbesondere in Kombination mit einer Reinigungsmittelart. Die Reinigungstemperatur kann hierbei einerseits hoch genug sein, um eine möglichst vollständige Entfernung der Verunreinigung zu gewährleisten und anderseits im Hinblick auf den Energieaufwand und eine Schonung der Textilie niedrig gehalten werden.

Unter einer Reinigungsvorrichtung wird insbesondere eine Waschmaschine, insbesondere automatische Haushaltswaschmaschine verstanden.

Die Wachmaschine kann in verschiedenen Ausgestaltungsformen vorliegen. Man unterscheidet Toplader, bei denen die Ladeluke an der Oberseite liegt, und Frontlader, bei denen ein Bullauge als Ladeluke an der Vorderseite dient. Vorteil des Topladers ist, dass die Abdichtung der Tür einfacher ausgeführt und die Trommel auf zwei Seiten durch Wälzlager abgestützt sein kann, ein Toplader lässt sich auch in sehr engen Räumen aufstellen, wo nicht genügend Platz zum Öffnen einer vorderen Tür zur Verfügung steht. Ein Frontlader hingegen bietet auf der Oberseite Platz für z. B. einen Wäschetrockner oder für eine Arbeitsfläche und wird deswegen gelegentlich anstelle eines Unterschranks in eine Küchenzeile eingebaut. Toplader sind nachteilig, da sie mehr Wasservolumen für die Wäschereinigung als Frontlader benötigen.

Die amerikanischen Toplader haben immer eine rotierende Trommel und Mischelemente (Agitator oder Discs), wobei sich die Mischelemente mit oder gegen die Trommeldrehrichtung bewegen können. Die Maschinen können eine Laugenumwälzung und Sprühvorrichtungen für die Lauge aufweisen. Grundsätzlich werden Deep Fill und HE-Toploader unterschieden. Deep Fill Toplader arbeiten mit vorgegebenen Wasserniveaus, haben also keine Beladungserkennung. HE Maschinen verfügen in der Regel über eine Beladungserkennung und steuern danach die Wassermengen. In der Regel haben die Maschinen keine eingebaute Heizung, sondern werden an Warmwasser angeschlossen.

Im Rahmen der vorliegenden Erfindung kann ein Parameter der Reinigungsstrategie einen bestimmten Typ einer solchen Reinigungsvorrichtung angeben. Denkbar ist auch, dass der Parameter zumindest teilweise manuell durchzuführende Reinigungsstrategien vorgibt, etwa eine Handwäsche. Auch kann der mindestens eine Parameter Einstellungen einer Reinigungsvorrichtung umfassen, beispielsweise ein Programm einer automatischen Haushaltswaschmaschine oder eine Sequenz solcher Programme.

Im Ergebnis kann mit dem mindestens einem Parameter der Reinigungsstrategie dem Benutzer die Entfernung der Verunreinigung erheblich erleichtert werden. Insbesondere bei Verunreinigungen, welche mit dem Auge nicht zu identifizieren sind, kann über das Verfahren eine im Hinblick auf die Reinigung, aber auch auf den Energieverbrauch und die Schonung des Materials der Textilie optimale Reinigungsstrategie empfohlen werden. Beispielsweise umfasst die Empfehlung der Reinigungsmittelart und der Einstellungen der Reinigungsvorrichtung, ob das Messergebnis einen gewissen Gehalt an Lipiden in der Verunreinigung anzeigt und daher entsprechende fettlösende Bestandteile im Reinigungsmittel enthalten sein sollen oder ob bestimmte Farbstoffe in der Verunreinigung vorhanden sind, welche über eine spezifische Reinigungsmittelart und Einstellungen der Reinigungsvorrichtung gezielt angegriffen werden können.

Der mindestens eine Parameter kann eine Empfehlung über eine Vorbehandlung der Textilie umfassen. Eine solche Vorbehandlung umfasst beispielsweise eine manuelle oder automatische Aufbringung eines Reinigungsmittels auf die Verunreinigung, insbesondere mit einer vorgegebenen Einwirkzeit, deren Dauer ebenfalls als Parameter der Reinigungsstrategie dienen kann. Anschließend kann die Textilie in einer Reinigungsvorrichtung, beispielsweise einer automatischen Haushaltswaschmaschine gereinigt werden.

Insbesondere umfasst das Verfahren weiterhin das Durchführen der Reinigungsstrategie über eine Reinigungsvorrichtung.

Hierbei kann das Messergebnis vor, während und/oder nach dem Durchführen der Reinigungsstrategie der Textilie erhalten werden. Mit einem Erhalten vor der Reinigung kann beispielsweise dem Benutzer vor einer durchzuführenden Reinigungsbehandlung eine Empfehlung über die zu verwendenden Reinigungsstrategie gegeben werden.

Bei einem Erhalten des Messergebnisses während der Reinigung kann die Reinigung beispielsweise dynamisch durchgeführt werden, d.h. eine Reinigungsvorrichtung kann sich während der Reinigung an die gerade ermittelte mindestens eine Ausgangsgröße anpassen, insbesondere indem die Ausgangsgröße kontinuierlich ermittelt wird. Beispielsweise passt eine Waschmaschine während des Waschprogramms beispielsweise die Temperatur oder die Reinigungsmittelmenge entsprechend der ermittelten Ausgangsgröße an. Hierbei kann insbesondere das Messergebnis von aus der Textilie gelösten Textilbestandteilen wie Textilfarbstoffen erhalten werden.

Mit einem Erhalten des Messergebnisses nach einer Reinigung kann beispielsweise das Ergebnis bzw. die Effektivität einer Reinigungsstrategie festgehalten und überprüft werden.

Die mindestens eine Ausgangsgröße kann dem Benutzer auf einer Anzeige ausgegeben werden bzw. eine entsprechende Ausgabe kann ausgelöst werden. Der Benutzer kann dann die Reinigungsstrategie durchführen. Alternativ oder zusätzlich hierzu kann in einer Ausgestaltung des Verfahrens die mindestens eine Ausgangsgröße an eine Reinigungsvorrichtung ausgegeben werden. Beispielsweise kann die mindestens eine Ausgangsgröße mindestens einen Parameter einer Reinigungsstrategie repräsentieren, welcher an die Reinigungsvorrichtung ausgegeben wird, so dass die Reinigungsvorrichtung beispielsweise die entsprechende Reinigungsstrategie als Voreinstellung übernimmt und der Benutzer die Reinigungsvorrichtung lediglich starten muss. Ebenso ist es denkbar, dass die Reinigungsvorrichtung mit der Ausgabe der mindestens einen Ausgangsgröße die Reinigungsstrategie automatisch durchführt. Die Reinigungsvorrichtung kann beispielsweise über eine Dosierungsvorrichtung für Reinigungsmittel verfügen, um die Reinigungsmittelart und Reinigungsmittelmenge entsprechend der empfohlenen Reinigungsstrategie automatisch bereitzustellen. Im Ergebnis wird hiermit die Benutzerfreundlichkeit des Verfahrens verbessert.

In einer Ausgestaltung des Verfahrens umfasst die Ermittlung der mindestens einen Ausgangsgröße einen Vergleich des Messergebnisses mit Vergleichswerten. Entsprechende Vergleichswerte können in einer Datenbank hinterlegt sein. Das Messergebnis kann einer Klassifizierung unterzogen werden, wobei die mindestens eine Ausgangsgröße durch ein Ergebnis der Klassifizierung erhalten oder beeinflusst wird. Eine Klassifizierung kann beispielsweise auf einem Vergleich des Messergebnisses mit einer Datenbank von bereits bekannten Messergebnissen beruhen.

Die Vergleichswerte bzw. eine hierfür vorgesehene Datenbank kann insbesondere Messergebnisse von typischen, in den Anwendungsgebieten der Textilien auftretenden Verunreinigungen enthalten. Beispielsweise kann im Haushaltsbereich auf Messergebnisse von typischen Verunreinigungen wie verschiedenen Nahrungsmittelresten, Spuren von Getränken, Gras oder Farben zurückgegriffen werden. Die Vergleichswerte können einen oder mehrerer Werte oder Wertebereiche umfassen. Weiter können den entsprechenden Vergleichswerten bestimmte Ausgangsgrößen zugeordnet sein, beispielsweise mindestens ein Parameter einer Reinigungsstrategie zur Entfernung der Verunreinigung.

In einer Ausgestaltung des Verfahrens umfasst das Verfahren weiterhin das Ermitteln des Messergebnisses über den mindestens einen Biosensor. Wie bereits eingangs ausgeführt, wird unter einem Biosensor ein Sensor verstanden, welcher zumindest ein biologisch aktives System zur Erzeugung des Messergebnisses umfasst.

Das Ermitteln des Messergebnisses durch den Biosensor kann insbesondere dadurch erfolgen, dass der Biosensor mit dem verunreinigten Textil im Bereich der Verunreinigung in Kontakt gebracht wird. Die auf der Textilie befindliche Verunreinigung kann insofern das Analyt darstellen. Beispielsweise wir ein sensitiver Bereich des Sensors mit der Verunreinigung in Kontakt gebracht. Ebenfalls ist es jedoch möglich, dass die Verunreinigung auf der Textilie zunächst mit einem Lösungsmittle (beispielsweise Wasser) benetzt wird und erst dann mit dem Biosensor in Kontakt gebracht wird.

Ein Lösungsmittel kann beispielsweise ein aprotisches (beispielsweise ein aprotisch-unpolares oder ein aprotisch-polares) Lösungsmittel oder ein protisches Lösungsmittel sein.

Ebenfalls ist möglich, dass der Biosensor nicht direkt mit der auf der Textilie befindlichen Verunreinigung in Kontakt gebracht zu werden braucht. Beispielsweise kann der Biosensor auch lediglich mit dem Lösungsmittel, welches zuvor in Kontakt mit der Verunreinigung der Textilie war, in Kontakt gebracht werden.

Dies kann insbesondere vorteilhaft sein, wenn sich eine Textilie bereits in der Reinigungsvorrichtung befindet und durch den Reinigungsvorgang eine Farbstoffausblutung erfolgt. Die Verunreinigung in Form des nicht fixierten Farbstoffs kann dann ein entsprechendes Messergebnis bei einem (beispielsweise frei in der Reinigungsvorrichtung beweglichen) Biosensor repräsentativ für die Verunreinigung ermöglichen. Dabei braucht der Biosensor lediglich in Kontakt mit dem Lösungsmittel in Form des Waschwassers zu gelangen.

In einer weiteren Ausgestaltung des Verfahrens umfasst das Verfahren weiterhin: Bestimmen eines Verunreinigungsprofils zumindest teilweise basierend auf der Ausgangsgröße, insbesondere basierend auf einer Mehrzahl von ermittelten Ausgangsgrößen, wobei die Ermittlung der mindestens einen Ausgangsgröße zumindest teilweise auf dem Verunreinigungsprofil basiert.

Über die mindestens eine Ausgangsgröße kann somit ein Verunreinigungsprofil erstellt werden, welches an die jeweilige Zusammensetzung der Verunreinigung angepasst wird. Insbesondere kann eine Mehrzahl von Ausgangsgrößen im Sinne einer Historie von ermittelten Ausgangsgrößen in ein Verunreinigungsprofil einfließen, so dass zukünftige Ermittlungen zumindest teilweise auf dem Verunreinigungsprofil basieren können. Damit kann die Ermittlung der mindestens einen Ausgangsgröße adaptiv gestaltet werden und sich über das Verunreinigungsprofil den jeweiligen Anforderungen genauer anpassen. Die Ermittlung der Ausgangsgröße kann insbesondere im Hinblick auf die Abhängigkeit von der chemischen Zusammensetzung der Verunreinigung genauer durchgeführt werden.

Beispielsweise kann ein Verunreinigungsprofil im Hinblick auf häufig vorkommende Zusammensetzungen von Verunreinigungen erstellt werden. Für die Ausgabe von mindestens einem Parameter einer Reinigungsstrategie können auch insbesondere die Reinigungsmittelart und der Reinigungsvorrichtungstyp in einem Verunreinigungsprofil berücksichtigt werden.

Denkbar ist es ebenfalls, dass eine Information über die Effektivität der Reinigungsstrategie in das Verunreinigungsprofil aufgenommen wird. Beispielsweise kann nach einem Reinigungsvorgang erneut ein Messergebnis ermittelt werden, um die Effektivität der Reinigungsstrategie zu ermitteln. Damit können zukünftige Reinigungsstrategien über das Verunreinigungsprofil weiter optimiert werden.

Ebenso kann der Benutzer nach der Reinigung eine Bewertung der mindestens einen Ausgangsgröße, beispielsweise eine Bewertung der Effektivität der Reinigungsstrategie vornehmen, welche in das Verunreinigungsprofil eingeht. Damit kann eine persönliche Anpassung der Ermittlung der Ausgangsgröße, insbesondere der Reinigungsstrategie erreicht werden.

Ebenfalls ist es möglich, dass die Informationen über die Effektivität und/oder die Bewertung des Benutzers für ein maschinelles Lernen genutzt wird. So kann das Verunreinigungsprofil beispielsweise zumindest teilweise basierend auf maschinellem Lernen bestimmt oder optimiert werden. Unter einem maschinellen Lernen wird insbesondere verstanden, dass ein künstliches System (zum Beispiel eine Vorrichtung gemäß dem zweiten Aspekt oder ein System gemäß dem dritten Aspekt) aus Beispielen lernt und diese nach Beendigung der Lernphase verallgemeinern kann. Das heißt, es werden nicht einfach die Beispiele auswendig gelernt, sondern es werden Muster und Gesetzmäßigkeiten in den Lerndaten erkannt. Hierzu können unterschiedliche Ansätze verfolgt werden. Beispielsweise kann ein überwachtes Lernen, ein teilüberwachtes Lernen, ein unüberwachtes Lernen, ein bestärktes Lernen und/oder ein Aktives Lernen eingesetzt werden. Ein überwachtes Lernen kann beispielsweise mittels eines künstlichen neuronalen Netzes (etwa einem rekurrenten neuronalen Netz) oder mittels einer Support Vector Machine erfolgen. Auch ein unüberwachtes Lernen kann beispielsweise mittels eines künstlichen neuronales Netzes (beispielsweis eines Autoencoders) erfolgen. Als Lerndaten dienen dann beispielsweise insbesondere erhaltene bzw. ermittelte Messergebnisse des zumindest einen Biosensors repräsentativ für eine Verunreinigung der Textilie und eine entsprechende Informationen über die Effektivität der Reinigung und/oder eine entsprechende Bewertung des Benutzers. Hierzu können auch Messergebnisse einer Vielzahl an Benutzern verwendet werden, um den Lernprozess zu weitere zu beschleunigen und zu verbessern.

Alternativ oder zusätzlich ist denkbar, dass erhaltene und/oder ermittelte Messergebnisse mit weiteren Informationen assoziiert werden, beispielsweise mit der Anzahl und/oder des jeweiligen Alters der Personen eines Haushalts zur Erstellung eines persönlichen Verbrauchsprofils oder beispielsweise mit der Jahreszeit zur Erstellung eines jahreszeitlichen Verbrauchsprofils.

Durch diese Maßnahmen kann die Zuverlässigkeit der Ermittlung der Ausgangsgröße weiter erhöht werden.

Gemäß einer bevorzugten Ausgestaltung des Verfahrens gemäß dem ersten Aspekt umfasst das Verfahren weiterhin ein Ermitteln einer Eigenschaftsinformation der Textilie, wobei das Ermitteln der zumindest einen Ausgangsgröße zusätzlich auf der ermittelten Eigenschaftsinformation der Textilie basiert.

Eine Eigenschaftsinformation der Textilie kann beispielsweise eine Information über das Material der Textilie umfassen. Beispielsweise ist die Eigenschaftsinformation indikativ für die Art des Materials der Textilie. Unter der Materialart wird insbesondere die Zusammensetzung zumindest eines Teils des Materials der Textilie verstanden. Beispielsweise ist die Eigenschaftsinformation indikativ für Naturfasern, Chemiefasern, Kunstfasern oder natürliche Materialien wie Baumwolle, Wolle, Seide oder Leder in der Textilie. Die Materialart hat ebenso erheblichen Einfluss auf eine optimale Behandlung der Textilie, beispielsweise eine Reinigungsbehandlung oder ein Bügeln. Eine Eigenschaftsinformation der Textilie kann ebenfalls repräsentativ für die Farbe des Materials der Textilie sein.

Eine Eigenschaftsinformation kann alternativ oder zusätzlich eine Strukturinformation über die Textilie umfassen. Beispielsweise ist die Strukturinformation indikativ für die Materialstruktur, Materialverteilung, den Materialverschleiß der Textilie oder eine Kombination hiervon.

Unter der Materialstruktur der Textilie wird insbesondere die Art und/oder Form der Textilie verstanden, beispielsweise ob es sich im ein Gewebe, eine Maschenware, einen Vliesstoff oder ein Faserflor handelt. Die entsprechende Strukturinformation kann insbesondere charakteristisch für die Art der Verflechtung von Fasern, wie diese beispielsweise über ein Weben, Wirken oder Stricken hergestellt wurde, oder charakteristisch für einen Vliesstoff sein. Ein Verflechtungsmuster bzw. ein Fadenverkreuzungsmuster und eine Fadenbindung können hierbei von der Strukturinformation umfasst werden. Fadendichte, Faserstärke, Faserlänge, Faserfeinheit und/oder Faserorientierung können insbesondere in der Strukturinformation erfasst sein. Die Materialstruktur der Textilie hat einen direkten Einfluss auf die Anforderungen an die Behandlung der Textilie, beispielsweise kann ein Vliessstoff andere Anforderungen an eine Reinigungsbehandlung stellen als eine gewirkte oder gewebte Struktur.

Mit der Materialverteilung der Textilie kann beispielsweise erfasst werden, ob die Textilie ein Mischgewebe aus unterschiedlichen Faserarten oder Fasermaterialien aufweist und/oder ob Teilbereiche der Textilie aus einem anderen Material gefertigt sind. Hierbei kann das Verhältnis der verschiedenen Materialien zueinander, beispielsweise ein Dichteverhältnis, Massenverhältnis oder Flächenverhältnis erfasst werden. Weiter in der Strukturinformation enthalten sein können die Art und Anzahl von Verbindungsstellen, beispielsweise Nähte, Verschweißungen oder Klebestellen.

Mit der Strukturinformation indikativ für den Materialverschleiß kann insbesondere festgehalten werden, ob sich Pillings, Risse, Löcher, Abnutzungen oder sonstige Strukturschäden an der Textilie befinden. Insbesondere für Pillings, welche durch ein Lösen von Fasern aus dem Textilverbund entstehen und in Form von Knoten an der Textiloberfläche auftreten, kann die Art, Form, Größe bzw. Höhe, Anzahl und/oder Verteilung des Materialverschleißes erfasst werden.

Durch die Ermittlung mindestens eines Behandlungsparameters basierend auf dem Materialverschleiß kann die Behandlung der Textilie entsprechend dem Materialverschleiß angepasst werden, um einen weiteren, verstärkten Materialverschleiß einzudämmen oder beispielsweise auch Pillings abzulösen um das Erscheinungsbild der Textilie wiederherzustellen. Das Ermitteln einer Eigenschaftsinformation der Textilie bevorzugt durch einen separaten Sensor, wie beispielsweise einen Struktursensor. Der Struktursensor kann hierbei beispielsweise Information über die Form, Beschaffenheit, Erscheinung und/oder Zusammensetzung der Textilie und des Materials der Textilie ermitteln. Der Struktursensor kann dabei beispielsweise ein optischer oder akustischer Sensor sein.

Der zumindest eine Biosensor weist zumindest ein aktives biologisches System, zumindest einen Signalumwandler und/oder zumindest ein elektronisches System auf. Seine Selektivität und Empfindlichkeit bezieht ein Biosensor insbesondere aus dem verwendeten biologischen System. Das biologische System ist ein immobilisiertes biologisches System. Unter einer Immobilisierung wird insbesondere die räumliche Fixierung von Bakterien, Zellen oder Enzymen in Gelpartikel, Kapseln oder auch in umgrenzte Reaktionsräume verstanden. Das biologische System kann auch als biologische Erfassungskomponente oder Biorezeptor bezeichnet werden. Das biologische System verwendet beispielsweise Biomoleküle von Organismen oder Rezeptoren, welche nach biologischen Systemen modelliert sind, um mit dem entsprechenden Analyt zu interagieren.

Beispielsweise basiert der Biosensor auf der direkten räumlichen Kopplung des biologisch aktiven Systems mit dem Signalumwandler (oder Transduktor) und dem elektronischen System. Durch die Wechselwirkung zwischen dem biologischen System und einem Analyt kann es zu physikochemischen Veränderungen kommen. Diese können mittels des Signalumwandlers, wie z. B. optoelektrischen Sensoren, amperometrischen oder potentiometrischen Elektroden oder speziellen Feldeffekttransistoren (chemisch sensitiver Feldeffekttransistor) bestimmt werden. Durch das elektronische System, welches insbesondere einen elektronischen Verstärker umfassen kann, kann das Signal des Signalumwandlers derart verstärkt werden, dass dies weiterverarbeitet werden kann.

Die Ermittlung eines Messergebnisses mittels eines Biosensors erfolgt somit insbesondere in den drei folgenden Schritten. Erstens erfolgt die Erkennung des Analyten durch das biologische System des Biosensors. Zweitens findet die Umwandlung der physikochemischen Veränderungen, die durch die Wechselwirkungen des Analyten mit dem biologischen System (beispielsweise einem Rezeptor) entstehen, in ein elektrisches Signal statt. Drittens wird das Signal dann verstärkt und/oder verarbeitet. Dabei können die Signalumwandlung und die Elektronik zur Verstärkung und/oder Verarbeitung kombiniert werden, z. B. in Mikrosensorsystemen.

Ein Signalumwandler des Biosensors kann beispielsweise optisch, piezoelektrisch, oder elektrochemisch basiert sein.

Das biologisch aktive System basiert auf Antikörpern, Enzymen, Organellen, Mikroorganismen, Nukleinsäuren, Zellen und/oder Gewebe.

Ein auf Antikörpern basierendes biologisch aktives System beruht insbesondere auf einer Antikörper/Antigen-Interaktion. Hierbei wird die (spezifische) Bindungsaffinität von Antikörpern für ein bestimmten Stoff oder ein Antigen ausgenutzt. Es hat sich gezeigt, dass auch eine derartige Bindung reversibel sein kann. Ein auf diesem Prinzip beruhender Biosensor hat voreilhaft eine hohe Spezifität.

Ein auf Enzymen basierendes biologisch aktives System beruht insbesondere auf einer enzymatischen Reaktion. Dabei kann die Erkennung des Analyts auf unterschiedliche Mechanismen erfolgen. Beispielsweise kann das Enzym das Analyt in ein detektierbares Produkt umwandeln. Beispielsweise kann eine Enzym-Hemmung oder -Aktivierung durch das Analyt detektiert werden. Beispielsweise kann eine Modifikation der Enzymeigenschaften durch die Interaktion mit dem Analyt detektiert werden. Ein auf Enzymen basiertes biologisch aktives System ist insbesondere deshalb vorteilhaft, da eine große Zahl von Reaktionen katalysiert werden kann, Gruppen von Analyten detektiert werden können und unterschiedliche Signalwandler verwendet werden können. Ebenfalls vorteilhaft ist, dass Enzyme während der Reaktion nicht verbraucht werden, sodass ein entsprechender Sensor problemlos mehrfach eingesetzt werden kann und für eine dauerhafte Verwendung geeignet ist.

Beispiele für Enzyme sind Amylasen, Proteasen (auch als Peptidasen bezeichnet) und/oder Lipasen. Ebenfalls können jedoch auch andere Hydrolasen, Esterasen und/oder Glykosidasen Anwendung finden.

Ein auf Nukleinsäuren basierendes biologisch aktives System basiert insbesondere auf Nukleinsäure-Interaktionen, insbesondere auf dem Prinzip der komplementären Basenpaare (Adenin:Thymin und Cytosin:Guanin) in der DNS.

Ein auf Organellen basierendes biologisch aktives System basiert insbesondere auf der Reaktion von Organellen auf bestimmte Stoffe. Derartige Systeme bzw. Sensoren konnten bereits zur Detektion einer Wasserverschmutzung eingesetzt werden. Analog können derartige Sensoren in Bezug auf die Verunreinigung einer Textilie eingesetzt werden.

Ein auf Zellen basierendes biologisch aktives System hat den Vorteil, dass eine große Bandbreite von Analyten detektiert werden kann. Zudem können entsprechende Sensoren aufgrund der in der Regel einfach handzuhabenden Zellen einfach hergestellt werden. Auch weisen derartige Sensoren einen vergleichsweise langen Aktivitätszeitraum auf.

Ein auf Gewebe basierendes biologisch aktives System ist vorteilhaft besonders einfach zu immobilisieren. Auch weist Gewebe eine vergleichsweise hohe Stabilität und Aktivität auf. Schließlich zeichnen sich entsprechende Sensoren auch durch eine gute Verfügbarkeit bei niedrigem Preis aus. Im Vergleich zu enzymbasierten Biosensoren entfällt zudem die Extraktion entsprechender Enzyme. Allerdings kann im Vergleich zu enzymbasierten Sensoren in der Regel nur eine geringere Spezifität erreicht werden.

Gemäß einer Ausgestaltung des Verfahrens gemäß dem ersten Aspekt basiert das biologisch aktive System auf einer Lipase-Reaktion, einer Amylase-Reaktion und/oder einer Protease-Reaktion.

Durch einen Biosensor dessen biologisch aktives System auf einer Lipase-Reaktion basiert, können insbesondere Lipide in der Zusammensetzung der Verunreinigung festgestellt werden. Dadurch kann insbesondere festgestellt werden, dass die Zusammensetzung der Verunreinigung Lipide enthält und/oder wie hoch der Anteil an Lipiden ist. Sind beispielsweise Lipide oder gewisse Gehalte an Lipiden in der Zusammensetzung der Verunreinigung vorhanden, kann die Verwendung von spezifischen Tensiden und/oder Lipasen in der empfohlenen Reinigungsstrategie enthalten sein.

Durch einen Biosensor dessen biologisch aktives System auf einer Amylase-Reaktion basiert, können insbesondere Polysaccharide in der Zusammensetzung der Verunreinigung festgestellt werden. Dadurch kann insbesondere festgestellt werden, dass die Zusammensetzung der Verunreinigung Polysaccharide enthält und/oder wie hoch der Anteil hieran ist. Sind beispielsweise Polysaccharide oder gewisse Gehalte an Polysacchariden in der Zusammensetzung der Verunreinigung vorhanden, kann die Verwendung von spezifischen Tensiden in der empfohlenen Reinigungsstrategie enthalten sein.

Durch einen Biosensor dessen biologisch aktives System auf einer Protease-Reaktion (auch Peptidase-Reaktion) basiert, können insbesondere Proteine in der Zusammensetzung der Verunreinigung festgestellt werden. Dadurch kann insbesondere festgestellt werden, dass die Zusammensetzung der Verunreinigung Proteine enthält und/oder wie hoch der Anteil hieran ist. Sind beispielsweise Proteine oder gewisse Gehalte an Proteinen in der Zusammensetzung der Verunreinigung vorhanden, kann die Verwendung von spezifischen Tensiden oder Bleichmitteln in der empfohlenen Reinigungsstrategie enthalten sein.

Gemäß einer Ausgestaltung des Verfahrens gemäß dem ersten Aspekt ist der zumindest eine Biosensor zur Detektion eines Lipids, eines Polysaccharids oder eines Proteins eingerichtet. Wie bereits ausgeführt, kann dies insbesondere mittels eines Biosensors mit einem biologisch aktiven System, welches auf Enzymen basiert, erfolgen. Allerdings sind auch Biosensoren mit einem anderen biologisch aktiven System verwendet werden. Beispiele für Lipide sind Fettsäuren, Triacylglyceride (Fette und fette Öle), Wachs, Phospholipide, Sphingolipide, Lipopolysaccharide und/oder Isoprenoide (Steroide, Carotinoide etc.). Beispiele für Polysaccharide sind Glykogen, Stärke (Amylose und Amylopektin), Pektine, Chitin, Callose und/oder Cellulose. Unter Proteinen werden insbesondere Eiweiße oder Eiweißstoffe verstanden. Durch einen oder mehrere Biosensoren, welche zur Detektion derartiger Stoffe eingerichtet sind, kann ein breites Spektrum der typischen Verunreinigungen von Textilien bestimmt werden und effektive Reinigungsstrategien vorgeschlagen oder durchgeführt werden.

Gemäß einer Ausgestaltung des Verfahrens gemäß dem ersten Aspekt ist der zumindest eine Biosensor ein spezifischer und/oder ein unspezifischer Biosensor. Unter einem spezifischen Biosensor wird ein Biosensor verstanden, welcher mit ausreichender Sicherheit lediglich einen bestimmen Stoff oder eine bestimmte Stoffgruppe detektiert. Unter einem unspezifischen Sensor wird ein Sensor verstanden, welcher mehrere Stoffe oder Stoffgruppen detektiert. Ein spezifischer Sensor hat demnach den Vorteil, dass bereits durch einen einzigen Sensor eine verlässliche Aussage in Bezug auf einen bestimmten Stoff der Zusammensetzung gemacht werden kann. Bei einem nicht spezifischen Sensor müssen unter Umständen mehrere Sensoren vorgesehen werden, um eine eindeutige Detektion eines bestimmten Stoffes zu ermöglichen.

Gemäß einer Ausgestaltung des Verfahrens gemäß dem ersten Aspekt ist eine Mehrzahl von Biosensoren zum Erhalten des Messergebnisses vorgesehen. Durch mehrere Biosensoren können vorteilhaft unterschiedliche Stoffe der Zusammensetzung der Verunreinigung ausgemacht werden. Sind mehrere Biosensoren vorgesehen, können beispielsweise alle spezifisch oder alle unspezifisch sein. Ebenfalls ist denkbar, dass die Mehrzahl von Biosensoren teilweise spezifisch und teilweise unspezifisch ist. Insbesondere in Bezug auf unspezifische Biosensoren ist es daher vorteilhaft, wenn mehrere Biosensoren vorgesehen sind.

Alternativ oder zusätzlich ist es ebenfalls denkbar, zumindest einen Biosensor mit einer Mehrzahl von sensitiven (spezifischen und/oder unspezifischen) Messbereichen zu versehen. Auf diese Weise kann auch bereits mittels eines Biosensors ein präziseres Messergebnis bezüglich der Verunreinigung erhalten werden.

In einer weiteren Ausgestaltung des Verfahrens ist mindestens eine der Vorrichtungen zur Durchführung des Verfahrens ein mobiles Gerät. Das mobile Gerät kann beispielsweise eine Sensorvorrichtung sein und den zumindest einen Biosensor aufweisen. Insbesondere kann eine Kommunikation über ein Kommunikationssystem zwischen dem mobilen Gerät, beispielsweise einem Smartphone, Laptop, Table einem Wearable oder einer Sensorvorrichtung, und mindestens einer weiteren Vorrichtung vorgenommen werden, beispielsweise einer Reinigungsvorrichtung und/oder einem Server. Gemäß einer Ausgestaltung umfasst die Vorrichtung gemäß dem zweiten Aspekt eine Kommunikationsschnittstelle. Beispielsweise ist die Kommunikationsschnittstelle für eine drahtgebundene oder drahtlose Kommunikation eingerichtet. Beispielsweise ist die Kommunikationsschnittstelle eine Netzwerkschnittstelle. Die Kommunikationsschnittstelle ist bevorzugt dazu eingerichtet mit einem Kommunikationssystem zu kommunizieren. Beispiele für ein Kommunikationssystem sind ein lokales Netzwerk (LAN), ein großräumiges Netzwerk (WAN), ein drahtloses Netzwerk (beispielsweise gemäß dem IEEE-802.11-Standard, dem Bluetooth (LE)-Standard und/oder dem NFC-Standard), ein drahtgebundenes Netzwerk, ein Mobilfunknetzwerk, ein Telefonnetzwerk und/oder das Internet. Ein Kommunikationssystem kann die Kommunikation mit einem externen Computer umfassen, beispielsweise über eine Internetverbindung.

Weitere Vorrichtungen können vorgesehen sein, beispielswiese ein Server und/oder beispielsweise ein Teil bzw. eine Komponente einer sogenannten Computer Cloud, welche Datenverarbeitungsressourcen dynamisch für verschiedene Nutzer in einem Kommunikationssystem bereitstellt. Unter einer Computer Cloud wird insbesondere eine Datenverarbeitungs-Infrastruktur gemäß der Definition des "National Institute for Standards and Technology" (NIST) für den englischen Begriff "Cloud Computing" verstanden. Ein Beispiel einer Computer Cloud ist eine Microsoft Windows Azure Platform.

In einer alternativen oder zusätzlichen Ausgestaltung kann eine Ermittlung des Messergebnisses über einen Biosensor vorgenommen werden, wobei der Biosensor an einer Reinigungsvorrichtung angeordnet ist. Beispielsweise ist der Biosensor zumindest teilweise an dem äußeren Gehäuse einer Reinigungsvorrichtung und außerhalb eines Reinigungsbehälters, in welchem eine Reinigung durchgeführt werden kann, angeordnet. Der Benutzer hat dadurch stets Zugang zu dem Biosensor, auch wenn die Reinigungsvorrichtung in Betrieb ist oder ausgeschaltet ist. Somit kann ein Verfahren gemäß dem ersten Aspekt jederzeit vorgenommen werden. Der Biosensor kann dabei an der Reinigungsvorrichtung fixiert sein, beispielsweise über Befestigungsmittel wie eine Verschraubung und/oder Verklebung. Ebenso kann der Biosensor auch über Positionierungsmittel frei positionierbar an der Reinigungsvorrichtung angeordnet sein, beispielsweise über eine magnetische Halterung.

Weiter kann der Biosensor zumindest teilweise im Inneren der Reinigungsvorrichtung, insbesondere im Bereich eines Reinigungsbehälters der Reinigungsvorrichtung, in welchem eine Reinigung durchgeführt werden kann, angeordnet sein. Insbesondere ist der Biosensor hierbei an einer dem Benutzer zugänglichen Position angeordnet. Der Biosensor kann auch hier an der Reinigungsvorrichtung fixiert sein, beispielsweise über Befestigungsmittel oder über Positionierungsmittel frei positionierbar sein. Der Biosensor ist beispielsweise an einer Öffnung des Reinigungsbehälters angeordnet, insbesondere an einer Tür. Bei einer Waschmaschine ist der Biosensor beispielsweise an der Ladeluke der Wäschetrommel angeordnet und/oder an der Dichtung der Ladeluke.

Ebenso kann der Biosensor frei beweglich im Inneren des Reinigungsbehälters ausgestaltet sein. In einer Ausgestaltung ist eine Sensorvorrichtung vorgesehen, wobei die Sensorvorrichtung umfasst: mindestens einen Biosensor zur Ermittlung des Messergebnisses, wobei die Vorrichtung dafür eingerichtet ist, während der Durchführung einer Reinigungsstrategie in einem Reinigungsbehälter einer Reinigungsvorrichtung das Messergebnis bereitzustellen. Beispielsweise ist die Sensorvorrichtung dafür eingerichtet, während eines Waschvorgangs frei beweglich in einer Wäschetrommel einer Waschmaschine angeordnet zu werden. Die Sensorvorrichtung kann eine für die Reinigungsbehandlung entsprechende Form aufweisen und beispielsweise eine abgerundete, insbesondere kugelige Form haben. Die Ermittlungsvorrichtung kann auch eine entsprechende Dichtigkeit und mechanische Beständigkeit aufweisen, so dass eine Waschlösung und auch aggressive Reinigungsmittel die Funktion der Sensorvorrichtung nicht beeinträchtigen. Die Sensorvorrichtung kann somit während eines Reinigungsvorgangs Messergebnisse der Verunreinigung bereitstellen, um die Reinigungsstrategie zu überwachen. Eine Ermittlung des Messergebnisses kann sowohl für eine Verunreinigung auf der Oberfläche einer Textilie vorgesehen sein und/oder auch Verunreinigungen wie gelöste Textilbestandteile wie Textilfarbstoffe umfassen, beispielsweise durch eine Untersuchung der Waschlösung.

Insbesondere wird mindestens ein Biosensor, welcher sich zumindest teilweise außerhalb des Reinigungsbehälters befindet, mit mindestens einem Biosensor, welcher sich zumindest teilweise innerhalb der Reinigungsvorrichtung bzw. innerhalb des Reinigungsbehälters befindet und welcher insbesondere frei beweglich ist, kombiniert. Mehrere Biosensoren gleicher oder unterschiedlicher Bauart können vorgesehen sein. Damit wird die Genauigkeit der Ermittlung der mindestens einen Ausgangsgröße verbessert, da die Biosensoren mit unterschiedlichen Positionen entsprechende Messergebnisse bereitstellen können. Insbesondere arbeitet zumindest einer der Biosensoren zumindest zeitweise kontinuierlich, so dass zu verschiedenen Zeitpunkten und insbesondere kontinuierlich während einer Reinigungsbehandlung Messergebnisse erhalten werden.

Ebenso kann mindestens ein Biosensor an einem Teil einer Reinigungsmittelverpackung angeordnet sein und beispielsweise in eine Verschlusskappe integriert oder auf einer Verschlussklappe anzuordnen sein, insbesondere durch ein Aufstecken. Damit ist der Biosensor frei und unabhängig von einer Reinigungsvorrichtung beweglich und kann vom Benutzer auf einfache Weise an einer Verunreinigung einer Textilie eingesetzt werden. Ebenso kann der Biosensor dann im Zusammenhang mit einer Vielzahl von verschiedenen Reinigungsvorrichtungen verwendet werden.

Gemäß dem zweiten Aspekt der Erfindung wird auch eine Vorrichtung beschrieben, umfassend zumindest einen Prozessor und zumindest einen Speicher mit Computerprogrammcode, wobei der zumindest eine Speicher und der Computerprogrammcode dazu eingerichtet sind, mit dem zumindest einen Prozessor zumindest ein Verfahren gemäß dem ersten Aspekt auszuführen und/oder zu steuern. Unter einem Prozessor soll zum Beispiel eine Kontrolleinheit, ein Mikroprozessor, eine Mikrokontrolleinheit wie ein Mikrocontroller, ein digitaler Signalprozessor (DSP), eine anwendungsspezifische Integrierte Schaltung (ASIC) oder ein Field Programmable Gate Arrays (FPGA) verstanden werden.

Zum Beispiel umfasst eine beispielhafte Vorrichtung ferner Mittel zum Speichern von Informationen wie einen Programmspeicher und/oder einen Hauptspeicher. Zum Beispiel umfasst eine beispielhafte erfindungsgemäße Vorrichtung ferner jeweils Mittel zum Empfangen und/oder Senden von Informationen über ein Netzwerk wie eine Netzwerkschnittstelle. Zum Beispiel sind beispielhafte erfindungsgemäße Vorrichtungen über ein oder mehrere Netzwerke miteinander verbunden und/oder verbindbar.

Eine beispielhafte Vorrichtung gemäß dem zweiten Aspekt ist oder umfasst etwa eine Datenverarbeitungsanlage, die softwaremäßig und/oder hardwaremäßig eingerichtet ist, um die jeweiligen Schritte eines beispielhaften Verfahrens gemäß dem zweiten Aspekt ausführen zu können. Beispiele für eine Datenverarbeitungsanlage sind ein Computer, ein Desktop-Computer, ein Server, ein Thinclient und/oder ein tragbarer Computer (Mobilgerät), wie etwa ein Laptop-Computer, ein Tablet-Computer, ein Wearable, ein persönlicher digitaler Assistent oder ein Smartphone.

Gemäß dem zweiten Aspekt der Erfindung wird auch ein Computerprogramm beschrieben, das Programmanweisungen umfasst, die einen Prozessor zur Ausführung und/oder Steuerung eines Verfahrens gemäß dem ersten Aspekt veranlassen, wenn das Computerprogramm auf dem Prozessor läuft. Ein beispielhaftes Programm gemäß der Erfindung kann in oder auf einem computerlesbaren Speichermedium gespeichert sein, welches eines oder mehrere Programme enthält.

Gemäß dem zweiten Aspekt der Erfindung wird auch ein computerlesbares Speichermedium beschrieben, welches ein Computerprogramm gemäß dem zweiten Aspekt enthält. Ein computerlesbares Speichermedium kann z.B. als magnetisches, elektrisches, elektromagnetisches, optisches und/oder andersartiges Speichermedium ausgebildet sein. Ein solches computerlesbares Speichermedium ist vorzugsweise gegenständlich (also "berührbar"), zum Beispiel ist es als Datenträgervorrichtung ausgebildet. Eine solche Datenträgervorrichtung ist beispielsweise tragbar oder in einer Vorrichtung fest installiert. Beispiele für eine solche Datenträgervorrichtung sind flüchtige oder nicht-flüchtige Speicher mit wahlfreiem-Zugriff (RAM) wie z.B. NOR-Flash-Speicher oder mit sequentiellen-Zugriff wie NAND-Flash-Speicher und/oder Speicher mit Nur-Lese-Zugriff (ROM) oder Schreib-Lese-Zugriff. Computerlesbar soll zum Beispiel so verstanden werden, dass das Speichermedium von einem Computer bzw. einer Datenverarbeitungsanlage (aus)gelesen und/oder beschrieben werden kann, beispielsweise von einem Prozessor.

Gemäß einem dritten Aspekt der Erfindung wird auch ein System beschrieben, umfassend mehrere Vorrichtungen, insbesondere ein mobiles Gerät (insbesondere eine Sensorvorrichtung) und eine Reinigungsvorrichtung, welche zusammen ein Verfahren gemäß dem ersten Aspekt durchführen.

Ein beispielhaftes System gemäß dem dritten Aspekt umfasst eine beispielhafte Reinigungsvorrichtung und zusätzlich eine weitere Vorrichtung, beispielsweise ein mobiles Gerät oder einen Server zur Durchführung eines beispielhaften Verfahrens gemäß dem ersten Aspekt.

Die zuvor in dieser Beschreibung beschriebenen beispielhaften Ausgestaltungen der vorliegenden Erfindung sollen auch in allen Kombinationen miteinander offenbart verstanden werden. Insbesondere sollen beispielhafte Ausgestaltungen in Bezug auf die unterschiedlichen Aspekten offenbart verstanden werden.

Insbesondere sollen durch die vorherige oder folgende Beschreibung von Verfahrensschritten gemäß bevorzugter Ausführungsformen eines Verfahrens auch entsprechende Mittel zur Durchführung der Verfahrensschritte durch bevorzugte Ausführungsformen einer Vorrichtung offenbart sein. Ebenfalls soll durch die Offenbarung von Mitteln einer Vorrichtung zur Durchführung eines Verfahrensschrittes auch der entsprechende Verfahrensschritt offenbart sein.

Weitere vorteilhafte beispielhafte Ausgestaltungen der Erfindung sind der folgenden detaillierten Beschreibung einiger beispielhafter Ausführungsformen der vorliegenden Erfindung, insbesondere in Verbindung mit den Figuren, zu entnehmen. Die Figuren sollen jedoch nur dem Zwecke der Verdeutlichung, nicht aber zur Bestimmung des Schutzbereiches der Erfindung dienen. Die Figuren sind nicht maßstabsgetreu und sollen lediglich das allgemeine Konzept der vorliegenden Erfindung beispielhaft widerspiegeln. Insbesondere sollen Merkmale, die in den Figuren enthalten sind, keineswegs als notwendiger Bestandteil der vorliegenden Erfindung erachtet werden.

### Kurze Beschreibung der Figuren

In der Zeichnung zeigt
- Fig. 1: ein Ablaufdiagramm eines Ausführungsbeispiels eines Verfahrens;
- Fig. 2: in einer schematischen Darstellung ein Ausführungsbeispiel eines Biosensors;
- Fig. 3: eine schematische Darstellung eines Ausführungsbeispiels einer Vorrichtung;
- Fig. 4: ein Blockdiagramm eines Ausführungsbeispiels einer Vorrichtung; und
- Fig. 5: unterschiedliche Ausführungsbeispiele eines Speichermediums.

### Detaillierte Beschreibung einiger beispielhafter Ausführungsformen der Erfindung

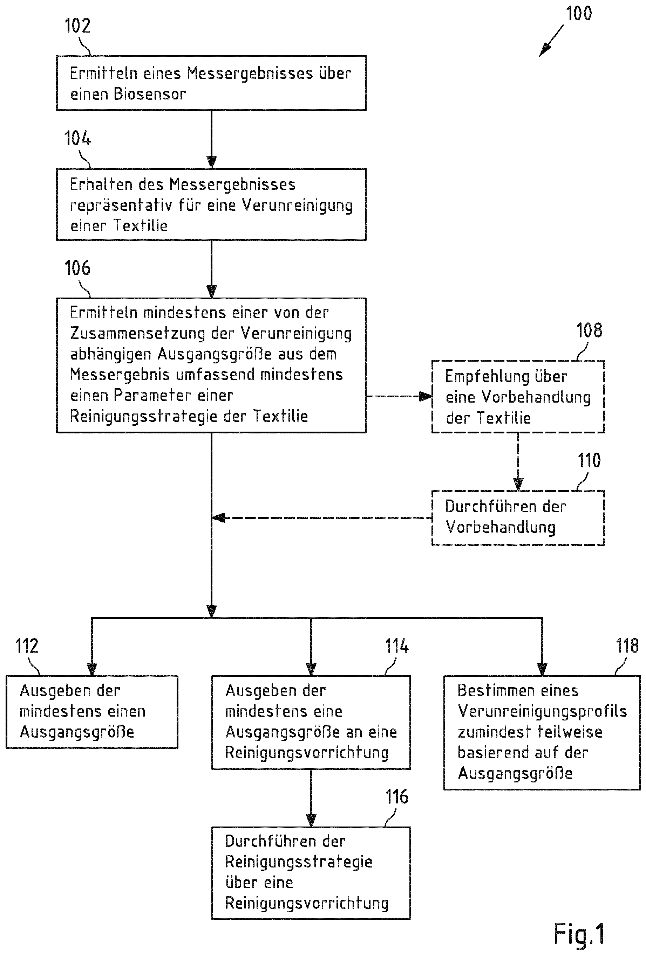

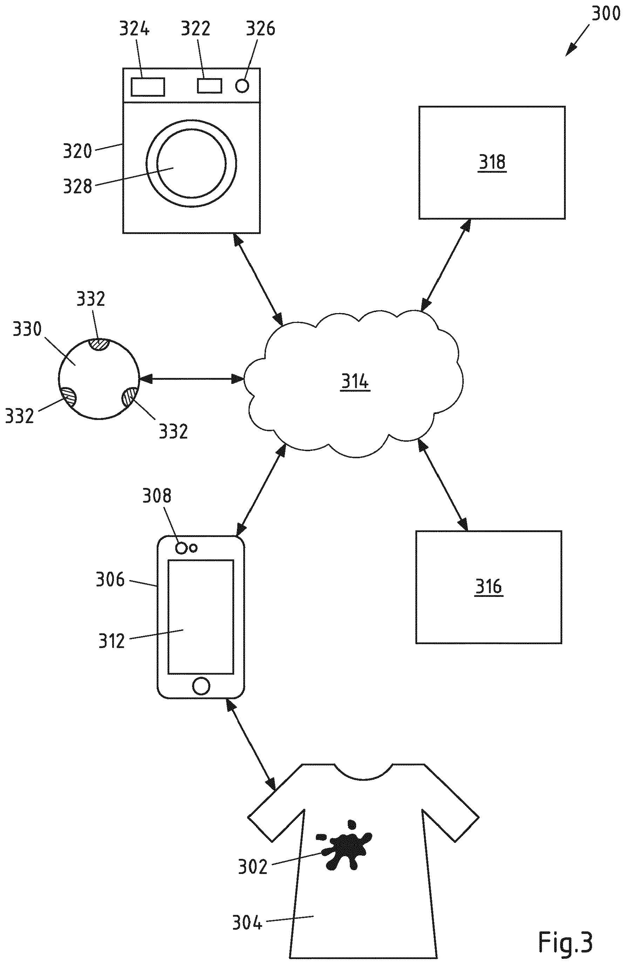

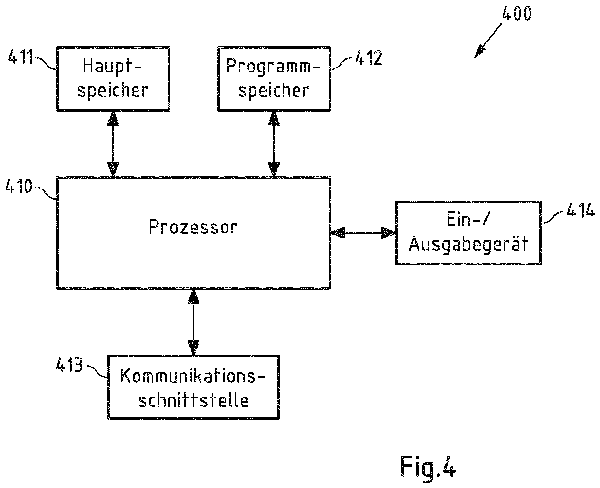

Fig.1 zeigt ein Ablaufdiagramm 100 eines Ausführungsbeispiels eines Verfahrens gemäß dem ersten Aspekt, welches durch eine Vorrichtung, beispielsweise eine der Vorrichtungen aus Fig. 3 und 4 durchgeführt wird.

In der Aktion 102 wird ein Messergebnis über einen Biosensor ermittelt, wobei das Messergebnis repräsentativ eine Verunreinigung einer Textilie (beispielsweise Verunreinigung 302 der Textile 304 aus Fig. 3) ist.

Dieses Messergebnis wird in Aktion 104 erhalten. Da das Messergebnis von der chemischen Zusammensetzung der Verunreinigung der Textilie abhängig ist, kann in Aktion 106 mindestens eine von der Zusammensetzung der Verunreinigung abhängige Ausgangsgröße aus dem Messergebnis ermittelt werden. Hierbei wird auf Grundlage der chemischen Zusammensetzung der Verunreinigung mindestens ein Parameter einer Reinigungsstrategie der Textilie ermittelt, wobei die Reinigungsstrategie eine Empfehlung zu einer optimalen Reinigung der Textilie von der Verunreinigung darstellt.

Zusätzlich kann optional mit Aktion 108 eine Empfehlung über eine Vorbehandlung der Textilie erfolgen. Bei bestimmten Zusammensetzungen der Verunreinigung kann eine entsprechende Vorbehandlung benötigt werden, welche beispielsweise von einer weiteren Vorrichtung in Aktion 110 durchgeführt wird.

In Aktion 112 wird eine Ausgabe der mindestens einen Ausgangsgröße veranlasst, beispielsweise eine Ausgabe auf einem Anzeigeelement, wobei dem Benutzer insbesondere Hinweise auf die Zusammensetzung der Verunreinigung und mindestens einen Parameter der Reinigungsstrategie angezeigt werden. Der Benutzer kann auf Grundlage der angezeigten Information bzw. Empfehlung eine Reinigung der Textilie durchführen.

Zudem oder alternativ kann in Aktion 114 eine Ausgabe der der mindestens einen Ausgangsgröße, insbesondere des mindestens einen Parameters der Reinigungsstrategie, an eine Reinigungsvorrichtung erfolgen. Die ausgegebenen Parameter der Reinigungsstrategie werden in Aktion 116 zur Durchführung einer Reinigung mittels der Reinigungsvorrichtung verwendet.

Zusätzlich kann in Aktion 118 ein Verunreinigungsprofil bestimmt werden, welches zumindest teilweise auf der Ausgangsgröße basiert. Damit kann die Ermittlung der mindestens einen Ausgangsgröße adaptiv gestaltet werden und sich über das Verunreinigungsprofil den jeweiligen Anforderungen genauer anpassen.

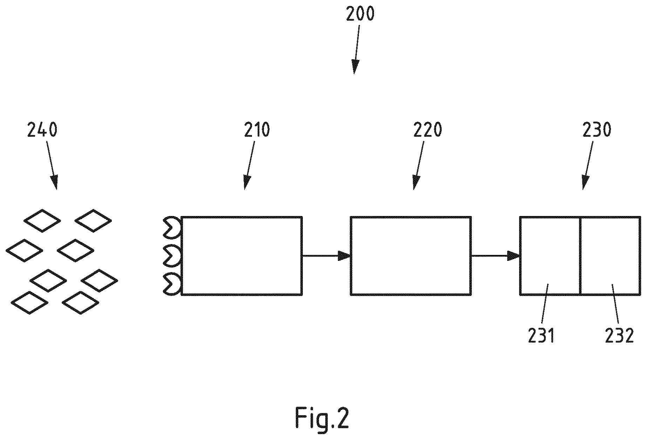

Fig. 2 zeigt in einer schematischen Darstellung ein Ausführungsbeispiel eines Biosensors 200. Der Biosensor umfasst ein biologisches aktives, immobilisiertes System 210, einen Signalumwandler (Transduktor) 220 und ein elektronisches System 230.

Das biologisch aktive, immobilisierte System 210 umfasst Biorezeptoren, welche beispielsweise Antikörper, Enzyme, Organellen, Mikroorganismen, Nukleinsäuren, Zellen und/oder Gewebe umfassen können. Durch die Biorezeptoren kann beispielweise eine Lipase-Reaktion, eine Amylase-Reaktion und/oder eine Protease-Reaktion in Anwesenheit des passenden Analyts 240 erfolgen.

Das Analyt 240 kann beispielweise die auf dem Textil befindliche Verunreinigung sein. Dabei kann das Analyt ebenfalls ein Lösungsmittel umfassen. Ebenfalls kann das Analyt unabhängig von dem Textil vorliegen und beispielsweise ein Lösungsmittel, welches Teile der Verunreinigung aufweist, umfassen. Beispielsweise kann das Analyt Waschwasser eines Reinigungsvorgangs einer Reinigungsvorrichtung sein, welches Spuren der Verunreinigung aufweist.

Der Signalumwandler 220 ist beispielsweise optisch, piezoelektrisch oder elektrochemisch basiert und umfasst beispielsweise einen optoelektrischen Sensor, amperometrische oder potentiometrische Elektroden oder speziellen Feldeffekttransistoren.

Das elektronische System 230 umfasst beispielsweise einen elektronischen Verstärker 231 und/oder eine Auswerteinheit 232. Mit der Auswerteeinheit kann beispielsweise das Messergebnis bereitgestellt werden. Ebenfalls ist denkbar, dass durch die Auswerteeinheit 231 bereits eine Ausgangsgröße ermittelt wird. Dies kann jedoch auch durch eine anderweitige Datenverarbeitungsanlage (beispielsweise Vorrichtung 316 aus Fig. 3) erfolgen.

Fig. 3 zeigt ein Ausführungsbeispiel einer Vorrichtung 300 gemäß dem zweiten Aspekt bzw. ein System gemäß dem dritten Aspekt. Die Vorrichtung 300 ist dazu eingerichtet bzw. umfasst entsprechende Mittel, ein Verfahren gemäß dem ersten Aspekt durchzuführen und/oder zu steuern.

Insbesondere ermöglicht die Vorrichtung 300 die Identifikation einer Zusammensetzung einer Verunreinigung 302 auf einer Textilie 304 und/oder gibt eine Empfehlung für eine Reinigungsstrategie zur Entfernung der Verunreinigung 302 von der Textilie 304.

Mit einem mobilen Gerät, hier einer Sensorvorrichtung 306, wird zunächst ein Messergebnis repräsentativ für eine Verunreinigung 302 ermittelt. Hierfür wird der Biosensor 308 verwendet. Die Sensorvorrichtung 306 verfügt zudem über ein Anzeigeelement 312.

Das ermittelte Messergebnis wird von einem Kommunikationssystem 314 erhalten. In Verbindung mit dem Kommunikationssystem 314 steht eine Datenverarbeitungsanlage 316, welche dafür eingerichtet ist, aus dem Messergebnis von der Zusammensetzung der Verunreinigung 302 abhängige Ausgangsgrößen zu ermitteln.

Die Ermittlung der Ausgangsgrößen umfasst hierbei einen Vergleich des Messergebnisses mit Vergleichswerten. Die Vergleichswerte sind in einer Datenbank 318 hinterlegt, welche ebenfalls in Kommunikation zu dem Kommunikationssystem 314 steht. Die Vergleichswerte der Datenbank 318 enthalten insbesondere Messergebnisse von typischen im Haushalt auftretenden Verunreinigungen. Weiter enthält die Datenbank 318 den Vergleichswerten zugeordnete Daten in Form einer chemischen Zusammensetzung und/oder Parametern in Bezug auf eine für die entsprechende Zusammensetzung optimale, zu empfehlende Reinigungsstrategie. Die Vorrichtungen 308, 3016 und/oder 318 können jedoch ebenfalls durch eine Vorrichtung realisiert werden.

Die Ausgangsgrößen umfassen Parameter einer solchen Reinigungsstrategie, wobei die Parameter eine Reinigungsmittelart, eine Reinigungsmittelmenge, eine Reinigungstemperatur, einen Reinigungsvorrichtungstyp und/oder Einstellungen einer Reinigungsvorrichtung 320 angeben. Diese Ausgangsgrößen können beispielsweise auf dem Anzeigeelement 312 der Sensorvorrichtung 306 angezeigt und somit dem Benutzer zur Verfügung gestellt werden. Dem Benutzer wird folglich eine Empfehlung über eine für die spezifische Verunreinigung 302 optimale Reinigungsstrategie zur Verfügung gestellt.

Die Reinigungsvorrichtung 320 steht ebenfalls in Kommunikation mit dem Kommunikationssystem 314, wodurch die Ausgangsgrößen an die Reinigungsvorrichtung 320 ausgegeben werden. Die Reinigungsvorrichtung 320 weist ein Anzeigeelement 322 auf, welches insbesondere die Ausgangsgrößen anzeigen kann. Weiter weist die Reinigungsvorrichtung 320 eine Dosierungsvorrichtung 324 für Reinigungsmittel auf. Die Dosierungsvorrichtung 324 kann hierbei ein Reinigungsmittel entsprechend den Parametern der Reinigungsstrategie in Bezug auf die Reinigungsmittelart und/oder die Reinigungsmittelmenge bereitstellen oder prüfen, ob das Reinigungsmittel entsprechend der empfohlenen Reinigungsstrategie in die Dosierungsvorrichtung 324 eingefüllt wurde.

Weiter weist die Reinigungsvorrichtung 320 ein Bedienelement 326 auf, welches die Steuerung der Reinigungsvorrichtung 320 durch einen Benutzer erlaubt. Die Reinigungsvorrichtung 320 übernimmt hierbei die Parameter der Reinigungsstrategie als Voreinstellung. Der Benutzer hat dann die Wahl, der Empfehlung der Reinigungsstrategie zu folgen und die Reinigungsvorrichtung 320 einfach über das Bedienelement 326 zu starten oder eine eigene, manuelle Einstellung der Reinigungsvorrichtung 320 über das Bedienelement 326 durchzuführen. Die Reinigung wird in einem Reinigungsbehälter 328, hier einer Wäschetrommel, durchgeführt.

Weiter ist in Fig. 3 eine alternative Sensorvorrichtung 330 gezeigt. Die Sensorvorrichtung 330 umfasst mehrere Sensoren 332. Die Sensorvorrichtung weist eine kugelige Form auf und ist dafür eingerichtet, während der Durchführung einer Reinigung in dem Reinigungsbehälter 328 angeordnet zu werden. Die Sensorvorrichtung 330 ist hierbei frei beweglich und gegen eine Einwirkung der Waschlösung in dem Reinigungsbehälter 328 beständig. Die Sensorvorrichtung 330 kann somit während eines Reinigungsvorgangs Messergebnisse der Verunreinigung 302 bereitstellen, um die Reinigungsstrategie zu überwachen. Die Sensorvorrichtung 330 kann auch Messergebnisse löslicher, nicht fixierte Textilfarbstoffe in der Waschlösung ermitteln. Damit kann ein Herauslösen der entsprechenden Textilfarbstoffe aus der Textilie 304 überwacht werden.

Fig. 4 zeigt ein Blockdiagramm eines Ausführungsbeispiels einer Vorrichtung 400, welche insbesondere ein beispielhaftes Verfahren gemäß dem ersten Aspekt ausführen kann. Die Vorrichtung 400 ist beispielsweise eine Vorrichtung gemäß dem zweiten oder ein System gemäß dem dritten Aspekt oder ein Teil hiervon.

Die Vorrichtung 400 kann insofern beispielsweise ein Computer, ein Desktop-Computer, ein Server, ein Thinclient oder ein tragbarer Computer (Mobilgerät), wie etwa ein Laptop-Computer, ein Tablet-Computer, ein persönlicher digitaler Assistent (PDA) oder ein Smartphone sein. Die Vorrichtung kann beispielsweise die Funktion eines Servers oder eines Clients erfüllen.

Prozessor 410 der Vorrichtung 400 ist insbesondere als Mikroprozessor, Mikrokontrolleinheit, Mikrocontroller, digitaler Signalprozessor (DSP), Anwendungsspezifische Integrierte Schaltung (ASIC) oder Field Programmable Gate Array (FPGA) ausgebildet.

Prozessor 410 führt Programmanweisungen aus, die in Programmspeicher 412 gespeichert sind, und speichert beispielsweise Zwischenergebnisse oder ähnliches in Arbeits- oder Hauptspeicher 411. Zum Beispiel ist Programmspeicher 412 ein nicht-flüchtiger Speicher wie ein Flash-Speicher, ein Magnetspeicher, ein EEPROM-Speicher (elektrisch löschbarer programmierbarer Nur-LeseSpeicher) und/oder ein optischer Speicher. Hauptspeicher 411 ist zum Beispiel ein flüchtiger oder nicht-flüchtiger Speicher, insbesondere ein Speicher mit wahlfreiem-Zugriff (RAM) wie ein statischer RAM-Speicher (SRAM), ein dynamischer RAM-Speicher (DRAM), ein ferroelektrischer RAM-Speicher (FeRAM) und/oder ein magnetischer RAM-Speicher (MRAM).

Programmspeicher 412 ist vorzugsweise ein lokaler mit der Vorrichtung 400 fest verbundener Datenträger. Mit der Vorrichtung 400 fest verbundene Datenträger sind beispielsweise Festplatten, die in die Vorrichtung 400 eingebaut sind. Alternativ kann der Datenträger beispielsweise auch ein mit der Vorrichtung 400 trennbar verbindbarer Datenträger sein wie ein Speicher-Stick, ein Wechseldatenträger, eine tragbare Festplatte, eine CD, eine DVD und/oder eine Diskette.

Programmspeicher 412 enthält beispielsweise das Betriebssystem von der Vorrichtung 400, das beim Starten der Vorrichtung 400 zumindest teilweise in Hauptspeicher 411 geladen und vom Prozessor 410 ausgeführt wird. Insbesondere wird beim Starten von Vorrichtung 400 zumindest ein Teil des Kerns des Betriebssystems in den Hauptspeicher 411 geladen und von Prozessor 410 ausgeführt. Das Betriebssystem von Vorrichtung 400 ist beispielsweise ein Windows -, UNIX-, Linux-, Android-, Apple iOS- und/oder MAC-Betriebssystem.

Das Betriebssystem ermöglicht insbesondere die Verwendung der Vorrichtung 400 zur Datenverarbeitung. Es verwaltet beispielsweise Betriebsmittel wie Hauptspeicher 411 und Programmspeicher 412, Netzwerkschnittstelle 413, Ein- und Ausgabegerät 414, stellt unter anderem durch Programmierschnittstellen anderen Programmen grundlegende Funktionen zur Verfügung und steuert die Ausführung von Programmen.

Prozessor 410 steuert die Kommunikationsschnittstelle 413, welche beispielsweise eine Netzwerkschnittstelle sein kann und als Netzwerkkarte, Netzwerkmodul und/oder Modem ausgebildet sein kann. Die Kommunikationsschnittstelle 413 ist insbesondere dazu eingerichtet, eine Verbindung der Vorrichtung 400 mit anderen Vorrichtungen, insbesondere über ein (drahtloses) Kommunikationssystem, beispielsweise ein Netzwerk, herzustellen und mit diesen zu kommunizieren. Die Kommunikationsschnittstelle 413 kann beispielsweise Daten (über das Kommunikationssystem) empfangen und an Prozessor 410 weiterleiten und/oder Daten von Prozessor 410 empfangen und (über das Kommunikationssystem) senden. Beispiele für ein Kommunikationssystem sind ein lokales Netzwerk (LAN), ein großräumiges Netzwerk (WAN), ein drahtloses Netzwerk (beispielsweise gemäß dem IEEE-802.11-Standard, dem Bluetooth (LE)-Standard und/oder dem NFC-Standard), ein drahtgebundenes Netzwerk, ein Mobilfunknetzwerk, ein Telefonnetzwerk und/oder das Internet.

Des Weiteren kann Prozessor 410 zumindest ein Ein-/Ausgabegerät 414 steuern. Ein-/Ausgabegerät 414 ist beispielsweise eine Tastatur, eine Maus, eine Anzeigeeinheit, ein Mikrofon, eine berührungsempfindliche Anzeigeeinheit, ein Lautsprecher, ein Lesegerät, ein Laufwerk und/oder eine Kamera. Ein-/Ausgabegerät 414 kann beispielsweise Eingaben eines Benutzers aufnehmen und an Prozessor 410 weiterleiten und/oder Informationen für den Benutzer von Prozessor 410 empfangen und ausgeben.

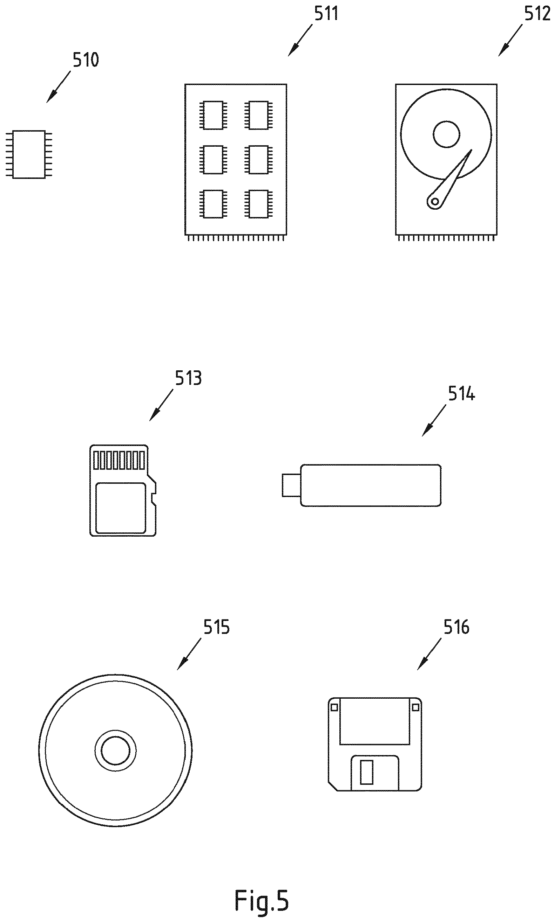

Fig.5 zeigt schließlich unterschiedliche Ausführungsbeispiele von Speichermedien, auf denen ein Ausführungsbeispiel eines erfindungsgemäßen Computerprogrammes gespeichert sein kann. Das Speichermedium kann beispielsweise ein magnetisches, elektrisches, optisches und/oder andersartiges Speichermedium sein. Das Speichermedium kann beispielsweise Teil eines Prozessors (z.B. des Prozessor 410 der Fig. 4) sein, beispielsweise ein (nicht-flüchtiger oder flüchtiger) Programmspeicher des Prozessors oder ein Teil davon (wie Programmspeicher 412 in Fig. 4). Ausführungsbeispiele eines Speichermediums sind ein Flash-Speicher 510, eine SSD-Festplatte 511, eine magnetische Festplatte 512, eine Speicherkarte 513, ein Memory Stick 514 (z.B. ein USB-Stick), eine CD-ROM oder DVD 515 oder eine Diskette 516.

Die in dieser Spezifikation beschriebenen Ausführungsbeispiele der vorliegenden Erfindung und die diesbezüglich jeweils angeführten optionalen Merkmale und Eigenschaften sollen auch in allen Kombinationen miteinander offenbart verstanden werden. Insbesondere soll auch die Beschreibung eines von einem Ausführungsbeispiel umfassten Merkmals - sofern nicht explizit gegenteilig erklärt - vorliegend nicht so verstanden werden, dass das Merkmal für die Funktion des Ausführungsbeispiels unerlässlich oder wesentlich ist. Die Abfolge der in dieser Spezifikation geschilderten Verfahrensschritte in den einzelnen Ablaufdiagrammen ist nicht zwingend, alternative Abfolgen der Verfahrensschritte sind denkbar. Die Verfahrensschritte können auf verschiedene Art und Weise implementiert werden, so ist eine Implementierung in Software (durch Programmanweisungen), Hardware oder eine Kombination von beidem zur Implementierung der Verfahrensschritte denkbar.

In den Patentansprüchen verwendete Begriffe wie "umfassen", "aufweisen", "beinhalten", "enthalten" und dergleichen schließen weitere Elemente oder Schritte nicht aus. Unter die Formulierung "zumindest teilweise" fallen sowohl der Fall "teilweise" als auch der Fall "vollständig". Die Formulierung "und/oder" soll dahingehend verstanden werden, dass sowohl die Alternative als auch die Kombination offenbart sein soll, also "A und/oder B" bedeutet "(A) oder (B) oder (A und B)". Die Verwendung des unbestimmten Artikels schließt eine Mehrzahl nicht aus. Eine einzelne Vorrichtung kann die Funktionen mehrerer in den Patentansprüchen genannten Einheiten bzw. Vorrichtungen ausführen. In den Patentansprüchen angegebene Bezugszeichen sind nicht als Beschränkungen der eingesetzten Mittel und Schritte anzusehen.

## Patentansprüche

1. Verfahren durchgeführt von einer Vorrichtung, umfassend:
- Erhalten eines Messergebnisses zumindest eines Biosensors (200) repräsentativ für eine Verunreinigung (302) einer Textilie (304), wobei der zumindest eine Biosensor (200)
i) zumindest ein biologisch aktives immobilisiertes System (210) aufweist, wobei das biologisch aktive immobilisierte System (210) auf Antikörpern, Enzymen, Organellen, Mikroorganismen, Nukleinsäuren, Zellen und/oder Gewebe basiert und,
ii)ein Signalumwandler (220) und/oder ein elektronisches System (230) aufweist; wobei der Signalumwandler eine physikochemische Veränderung durch eine Wechselwirkung zwischen dem biologischen aktiven immobilisierten System und einem Analyt bestimmt;
- Ermitteln mindestens einer von der Zusammensetzung der Verunreinigung (302) abhängigen Ausgangsgröße auf Basis des Messergebnisses; und
- Ausgeben oder Auslösen eines Ausgebens der mindestens einen Ausgangsgröße.

2. Verfahren nach Anspruch 1, wobei die Verunreinigung (302) eine lokalisierbare oder eine nicht lokalisierbare Verunreinigung ist.

3. Verfahren nach Anspruch 1 oder 2, wobei die mindestens eine Ausgangsgröße mindestens einen Parameter einer Reinigungsstrategie der Textilie (304) umfasst.

4. Verfahren nach Anspruch 3, wobei der mindestens eine Parameter der Reinigungsstrategie eine Reinigungsmittelart, eine Reinigungsmittelmenge, eine Reinigungstemperatur, einen Reinigungsvorrichtungstyp, Einstellungen einer Reinigungsvorrichtung (320) oder Kombinationen hiervon repräsentiert.

5. Verfahren nach Anspruch 3 oder 4, wobei der mindestens eine Parameter der Reinigungsstrategie eine Empfehlung über eine Vorbehandlung der Verunreinigung (204, 302) umfasst.

6. Verfahren nach einem der Ansprüche 1 bis 5, wobei die Ermittlung der mindestens einen Ausgangsgröße einen Vergleich des Messergebnisses mit Vergleichswerten umfasst.

7. Verfahren nach einem der Ansprüche 1 bis 6, das Verfahren weiterhin umfassend:
- Bestimmen eines Verunreinigungsprofils zumindest teilweise basierend auf der Ausgangsgröße, insbesondere basierend auf einer Mehrzahl von ermittelten Ausgangsgrößen,
- wobei die Ermittlung der mindestens einen Ausgangsgröße zumindest teilweise auf dem Verunreinigungsprofil basiert.

8. Verfahren nach einem der Ansprüche 1 bis 7, das Verfahren weiterhin umfassend:
- Ermitteln einer Eigenschaftsinformation der Textilie (304), wobei das Ermitteln der zumindest einen Ausgangsgröße zusätzlich auf der ermittelten Eigenschaftsinformation der Textilie (304) basiert.

9. Verfahren nach Anspruch 8, wobei das biologisch aktive System (210) auf einer Lipase-Reaktion, einer Amylase-Reaktion und/oder einer Protease-Reaktion basiert.

10. Verfahren nach einem der Ansprüche 1 bis 9, wobei der zumindest eine Biosensor (200) zur Detektion eines Lipids, eines Polysaccharids oder eines Proteins eingerichtet ist.

11. Verfahren nach einem der Ansprüche 1 bis 10, wobei der zumindest eine Biosensor (200) ein spezifischer und/oder ein unspezifischer Biosensor ist.

12. Verfahren nach einem der Ansprüche 1 bis 11, wobei eine Mehrzahl von Biosensoren zum Erhalten des Messergebnisses vorgesehen ist.

13. Vorrichtung, umfassend zumindest einen Prozessor und zumindest einen Speicher mit Computerprogrammcode welche dazu eingerichtet sind, ein Verfahren nach einem der Ansprüche 1 bis 13 durchzuführen und/oder zu steuern.

14. Computerprogrammprodukt, das Programmanweisungen umfasst, die dazu eingerichtet sind, einen Prozessor (410) der Vorrichtung nach Anspruch 13 zur Ausführung und/oder Steuerung eines Verfahrens nach einem der Ansprüche 1 bis 12 zu veranlassen, wenn das Computerprogramm auf dem Prozessor (410) der Vorrichtung nach Anspruch 13 läuft.

## Claims

1. A method carried out by a device, comprising:
- obtaining a measurement result of at least one biosensor (200), which is representative of soiling (302) of a textile (304), wherein the at least one biosensor (200) has i) at least one biologically active immobilized system (210), wherein the biologically active immobilized system (210) is based on antibodies, enzymes, organelles, microorganisms, nucleic acids, cells and/or tissue, and ii) a signal converter (220) and/or an electronic system (230); wherein the signal converter determines a physicochemical change resulting from an interaction between the biological active immobilized system and an analyte;
- determining at least one output variable, which is dependent on the composition of the soiling (302), on the basis of the measurement result; and
- outputting or triggering the output of the at least one output variable.

2. The method according to claim 1, wherein the soiling (302) is locatable or non-locatable soiling.

3. The method according to claim 1 or 2, wherein the at least one output variable comprises at least one parameter of a cleaning strategy of the textile (304).

4. The method according to claim 3, wherein the at least one parameter of the cleaning strategy constitutes a type of cleaning agent, a quantity of cleaning agent, a cleaning temperature, a type of cleaning device, settings of a cleaning device (320) or combinations thereof.

5. The method according to claim 3 or 4, wherein the at least one parameter of the cleaning strategy includes a recommendation on pretreatment of the soiling (204, 302).

6. The method according to one of claims 1 to 5, wherein the determination of the at least one output variable includes comparing the measurement result with comparative values.

7. The method according to one of claims 1 to 6, the method further comprising:
- determining a soiling profile based at least in part on the output variable, in particular based on a plurality of determined output variables,
- wherein the determination of the at least one output variable is based at least in part on the soiling profile.

8. The method according to one of claims 1 to 7, the method further comprising:
- determining property information for the textile (304), wherein the determination of the at least one output variable is additionally based on the determined property information for the textile (304).

9. The method according to claim 8, wherein the biologically active system (210) is based on a lipase reaction, an amylase reaction, and/or a protease reaction.

10. The method according to one of claims 1 to 9, wherein the at least one biosensor (200) is configured to detect a lipid, a polysaccharide or a protein.

11. The method according to one of claims 1 to 10, wherein the at least one biosensor (200) is a specific and/or a non-specific biosensor.

12. The method according to one of claims 1 to 11, wherein a plurality of biosensors is provided for obtaining the measurement result.

13. A device comprising at least one processor and at least one memory having computer program code which are configured to carry out and/or control a method according to one of claims 1 to 13.

14. A computer program product comprising program instructions which are configured to cause a processor (410) of the device according to claim 13 to carry out and/or control a method according to one of claims 1 to 12 when the computer program runs on the processor (410) of the device according to claim 13.

## Revendications

1. Procédé mis en oeuvre par un dispositif, comprenant :
- l'obtention d'un résultat de mesure d'au moins un biocapteur (200), lequel résultat de mesure représente un encrassement (302) d'un textile (304), dans lequel l'au moins un biocapteur (200) i) présente au moins un système immobilisé biologiquement actif (210), dans lequel le système immobilisé biologiquement actif (210) est basé sur des anticorps, des enzymes, des organites, des microorganismes, des acides nucléiques, des cellules et/ou des tissus, et ii) présente un convertisseur de signal (220) et/ou un système électronique (230) ; dans lequel le convertisseur de signal définit une modification physico-chimique causée par une interaction entre le système immobilisé biologiquement actif et un analyte ;
- la détermination d'au moins une grandeur de sortie dépendant de la composition de l'encrassement (302) sur la base du résultat de mesure ; et
- la sortie ou le déclenchement d'une sortie de l'au moins une grandeur de sortie.

2. Procédé selon la revendication 1, dans lequel l'encrassement (302) est un encrassement localisable ou un encrassement non localisable.

3. Procédé selon la revendication 1 ou 2, dans lequel l'au moins une grandeur de sortie comprend au moins un paramètre d'une stratégie de nettoyage du textile (304).

4. Procédé selon la revendication 3, dans lequel l'au moins un paramètre de la stratégie de nettoyage représente un type d'agent de nettoyage, une quantité d'agent de nettoyage, une température de nettoyage, un type de dispositif de nettoyage, des réglages d'un dispositif de nettoyage (320) ou des combinaisons de ceux-ci.

5. Procédé selon la revendication 3 ou 4, dans lequel l'au moins un paramètre de la stratégie de nettoyage comprend une recommandation concernant un prétraitement de l'encrassement (204, 302).

6. Procédé selon l'une des revendications 1 à 5, dans lequel la détermination de l'au moins une grandeur de sortie comprend une comparaison du résultat de mesure avec des valeurs de comparaison.

7. Procédé selon l'une des revendications 1 à 6, le procédé comprenant en outre :
- la définition d'un profil d'encrassement au moins en partie sur la base de la grandeur de sortie, en particulier sur la base d'une pluralité de grandeurs de sortie déterminées,
- dans lequel la détermination de l'au moins une grandeur de sortie est basée au moins en partie sur le profil d'encrassement.

8. Procédé selon l'une des revendications 1 à 7, le procédé comprenant en outre :
- la détermination d'une information de propriété du textile (304), dans lequel la détermination de l'au moins une grandeur de sortie est en outre basée sur l'information de propriété déterminée du textile (304).

9. Procédé selon la revendication 8, dans lequel le système biologiquement actif (210) est basé sur une réaction de la lipase, une réaction de l'amylase et/ou une réaction de la protéase.

10. Procédé selon l'une des revendications 1 à 9, dans lequel l'au moins un biocapteur (200) est configuré pour la détection d'un lipide, d'un polysaccharide ou d'une protéine.

11. Procédé selon l'une des revendications 1 à 10, dans lequel l'au moins un biocapteur (200) est un biocapteur spécifique et/ou un biocapteur non spécifique.

12. Procédé selon l'une des revendications 1 à 11, dans lequel une pluralité de biocapteurs est prévue pour l'obtention du résultat de mesure.

13. Dispositif comprenant au moins un processeur et au moins une mémoire comportant un code de programme informatique, lesquels sont configurés pour mettre en oeuvre et/ou pour commander un procédé selon l'une des revendications 1 à 13.

14. Produit programme informatique comprenant des instructions de programme qui sont configurées pour amener un processeur (410) du dispositif selon la revendication 13 à réaliser et/ou à commander un procédé selon l'une des revendications 1 à 12 lorsque le programme informatique est exécuté sur le processeur (410) du dispositif selon la revendication 13.
